Europäisches Patentamt

European Patent Office

Office européen des brevets

(19)

(11) **EP 1 258 522 A2**

(12) **EUROPEAN PATENT APPLICATION**

(43) Date of publication:
20.11.2002 Bulletin 2002/47

(51) Int Cl.7: **C09K 15/02**

(21) Application number: **02012889.8**

(22) Date of filing: **31.03.1993**

(84) Designated Contracting States:
**CH DE ES FR GB IT LI**

(30) Priority: **03.04.1992 US 863056**

(62) Document number(s) of the earlier application(s) in
accordance with Art. 76 EPC:
**93908878.7 / 0 596 059**

(71) Applicant: **L'air Liquide, S.A. à Directoire et
Conseil de Surveillance pour l'Etude et
l'Exploitation des Procédés Georges Claude
75321 Paris Cedex 07 (FR)**

(72) Inventor: **Spencer, Kevin
Hinsdale, IL 60521 (US)**

(74) Representative: **Mellul-Bendelac, Sylvie et al
L'Air Liquide,
Service Propriété Intellectuelle,
75, Quai d'Orsay
75321 Paris Cedex 07 (FR)**

Remarks:
This application was filed on 11 - 06 - 2002 as a
divisional application to the application mentioned
under INID code 62.

(54) **A method of controlling the oxidation of chemicals during storage using noble gases**

(57)    A method of controlling oxidation of a chemical or chemical preparation during storage, which entails contacting the chemicals or chemical preparations with at least one noble gas, a mixture of noble gases or a gaseous mixture containing at least one noble gas during at least a portion of storage.

FIG._1

## Description

BACKGROUND OF THE INVENTION

Field of the Invention:

The present invention provides a method of controlling the oxidation of chemicals during storage using a noble gas, a mixture of noble gases or a gaseous mixture containing at least one noble gas.

Description of the Background:

Conventional methods of controlling the oxidation of chemicals during storage have merely relied upon the use of different gases to physically and inertly displace oxygen.

For example, French Patent 1,339,669 discloses that argon may be used to render oxidizable chemicals of biological origin inert to oxidation because it desorbs oxygen. Thus, this patent specifically states that oxygen must be removed for any inhibitory effect upon oxidation to be observed. Similarly, in U.S. 3,143,471, a method is described whereby dry argon is used in the packaging of lyophilized pharmaceuticals, generally, wherein the sole object of the process is the displacement of water and oxygen from the packaging space.

In fact, it is well established that the preferred method for the storage of chemicals or chemical preparations is under an inert or non-reactive atmosphere nitrogen is usually used due to cost considerations.

For example, inert or non-reactive atmospheres which can be either nitrogen or argon are recommended for packaging or storing chemicals or chemical preparations such as oils (JP 3200568), polyacrylic acids (U.S. 4,622,425), N,N-dialkylaminopropyl compounds (JP 63077848), alpha-cyano-acrylic ester (JP 52097913, 85007609), vinylidene halide compounds (U.S. 3,957,892), sodium products (FR 2,261,518), chemical propellants (DE 3,007,712), medical solutions (U.S. 4,664,256), silicon wafers (U.S. 4,450,960), pharmaceuticals such as ergopeptide alkaloids in alcohol solution (U.S. 4,138,565), dihydroxycholecalciferol (U.S. 4,308,264, GB 2,091,556, DE 3,202,622, FR 2,498,450, JP 57144218, GB 2,091,556, CA 1,174,605, IL 64798, CH 651208, JP 86044845, DE 3,202,622), pharmaceutical preparations from biological sources such as extracts and vaccines (U.S. 3,143,471, GB 1006163, DE 1,753,586, DE 2,208,321, NL 7,210,592, BE 786991, JP 4823919, FR 2,194,418, GB 1,376,362, CA 989311), chlorophyll (JP 60056984, JP 88005034), proteins (EP 204532, JP 61278764, U. S. 4,644,976, U.S. 4,678,754, CA 1,263,075, EP 204532, DE 3,680954), enzymes such as urokinase or streptokinase (JP 54129185, 8700559), or liposome components (U.S. 4,830,858), cells (SU 1289437), tissues and organs (BE 8318889, NL 7509357, DE 2,437,812, SE 7,508,725, FR 2,281,062, GB 1,466,205, U.S. 4,008,754, CH 589403, AT 7505161, DE 2,437,812, IT 1040412, JP 51035583, JP 83040521), developer sheets (JP 3057689, U.S. 5,064,070), photosensitive donor media (U.S. 4,965,165), agricultural antiseptic suspensions (JP 2104502).

Further, similar processes exist in which efficient removal of oxygen by other means can substitute sufficiently for inert gas use, including use of rare gases, such as in lyophilization of pharmaceuticals of biological origin, for example vaccines or extracts (U.S. 3,378,443), or by using a chemical absorber of oxygen (JP 52086987).

Additionally, preparation or synthesis of chemicals under generally inert atmospheres in which the use of either nitrogen or argon without distinction is recommended include, as examples, purification and stabilization of catechol-containing proteins (U.S. 4,496,397), preparation of cosmetic dyes (U.S. 4,314,810), synthesis of peptides (FR 1,454,653, AU 6565762), synthesis of antibiotics (JP 58039650, JP 89039416).

However, despite the extensive literature relating to the use of non-reactive gases in the storage of chemicals or chemical preparations, no specific description or suggestion has yet been made in any of this literature regarding the use of any gases to specifically control oxidation reactions. In fact, it would be extremely desirable if a gas or gaseous mixture were known which could specifically and directly control oxidation reactions.

SUMMARY OF THE INVENTION

Accordingly, it is an object of the present invention to provide a method for controlling the oxidation of chemicals or chemical preparations during storage thereof.

It is also an object of the present invention to provide a method for inhibiting the oxidation of chemicals or chemical preparations during storage thereof.

It is, further, an object of the present invention to provide a method for enhancing the stability of chemicals or chemical preparations during storage thereof.

Accordingly, the above objects and others which will become more apparent in view of the following disclosure are provided by a method of controlling the oxidation of chemicals or chemical preparations during at least a portion of storage thereof by contacting the same with a noble gas, a mixture of noble gases or a gaseous mixture containing at least one noble gas.

BRIEF DESCRIPTION OF THE DRAWINGS

Figure 1 represents the absorptivity of a solution of Quercetin under both oxygen and nitrogen atmosphere.

Figure 2 illustrates the absorptivity of Quercetin under different atmospheres of oxygen, nitrogen, argon, krypton and xenon at 25°C.

Figure 3 illustrates the absorptivity of Quercetin under different atmospheres of oxygen, nitrogen, argon, neon and air at 25°C.

Figure 4 illustrates the absorptivity of morphine sulfate under different atmospheres of oxygen, nitrogen and argon after 31 days of storage at ambient temperature.

Figure 5 illustrates the absorptivity of apomorphine hydrochloride under different atmospheres of oxygen, nitrogen and argon after 31 days of storage at ambient temperature.

Figure 6 illustrates the absorptivity of apomorphine hydrochloride under different atmospheres of oxygen, nitrogen and argon after 31 days of storage at ambient temperature.

Figure 7 illustrates the absorptivity of apomorphine hydrochloride under different atmospheres of nitrogen and argon after 31 days at ambient temperature.

Figure 8 illustrates the absorptivity of epinephrine hydrochloride under different atmospheres of nitrogen, argon, krypton and xenon after 31 days at ambient temperature.

Figure 9 illustrates the absorptivity of epinephrine hydrochloride under different atmospheres of nitrogen and xenon for 31 days and three and one-half months, respectively, for each.

Figure 10 illustrates the absorptivity of L-ascorbic acid under different atmospheres of oxygen and nitrogen.

Figure 11 illustrates the absorptivity of L-ascorbic acid under different atmospheres of nitrogen, argon, krypton, xenon and neon after 30 days at ambient temperature.

Figure 12 illustrates the absorptivity of retinol under different atmospheres of nitrogen, argon, krypton and xenon after 3 months at ambient temperature in darkness.

Figure 13 illustrates the absorptivity of tetracycline hydrochloride under different atmospheres of air and nitrogen for 33 days and four months each, respectively.

Figure 14 illustrates the absorptivity of tetracycline hydrochloride under different atmospheres of air and neon for 33 days and four months each, respectively.

DETAILED DESCRIPTION OF THE PREFERRED EMBODIMENTS

Generally, in accordance with the present invention, it has been surprisingly discovered that it is possible to directly affect the rate of oxidation of chemicals or chemical preparations. That is, in accordance with the present invention it has been discovered that certain gases can directly affect the oxidation rate of chemicals and not merely slow oxidation only as function of physical and inert displacement of oxygen. Generally, the present invention is effected by contacting the chemicals or chemical preparations during at least a portion of storage thereof to a noble gas, a mixture of noble gases or a gaseous mixture containing at least one noble gas.

Thus, in general, the present invention is predicated upon the discovery that by contacting chemicals or chemical preparations with an atmosphere containing a noble gas, a mixture of noble or a gaseous mixture containing at least one noble gas during at least a portion of storage it is possible to effectively control oxidation reactions which degrade such compounds or preparations. Further, in accordance with the present invention, it has specifically been discovered that noble gases are surprisingly more effective than nitrogen or other inert or non-reactive gases in actually and effectively controlling the oxidation of chemical compounds or chemical preparations. Hence, the present invention is predicated upon a, heretofore, undiscovered property of noble gases in interfering with oxidation reactions of chemicals and chemical preparations during storage.

In accordance with the present invention, a method is also provided for inhibiting oxidation of chemicals or chemical preparations. Further, the present invention may be advantageously used at any time during storage of chemicals or chemical preparations.

Many chemicals or chemical preparations are currently packaged or otherwise stored under an inert atmosphere to prevent oxidation. This storage is essential to protect the value of the chemicals or chemical preparations. At present, gases used in composing such atmospheres are considered equivalent, and nitrogen is preferentially used due to its low cost. This use is, however, limited to the ability of nitrogen to displace oxygen.

By contrast, it has been surprisingly discovered in accordance with the present invention that noble gases possess a surprising ability to control, and specifically inhibit oxidation to a degree quite superior to that exhibited by nitrogen. In general, it has been discovered that the use of noble gases instead of nitrogen confers additional stability to stored chemical products or chemical preparations.

As used herein, the term "noble gas" generally includes argon, xenon, krypton or neon. While other noble gases, such as radon or helium may be used, the use of radon is generally impractical as it is dangerously reactive. While helium confers a positive benefit, and less than the other noble gases, and may be freely admixed with any other noble gas to advantage, its use is limited by its poor solubility in aqueous solutions and its tendency to dissipate by penetrating packaging materials. However, it is reiterated that the present invention is predicated upon the surprising discovery of the ability of all of the noble gases to control the oxidation of chemicals or chemical preparations.

Furthermore, in accordance with the present invention, it has been found that atmospheres containing noble gases in quantities greater than those normally found in ambient air significantly improve storage characteristics and shelf-life of chemicals or chemical preparations. Further, the effective improvement is of wide practical utility and clear economic benefit.

Generally, any amount of noble gas above that found in normal atmospheric air may be used to advantage in the present invention. For example, argon is generally accepted as being present in the atmosphere in the amount of about 0.934% by volume. While neon, krypton and xenon are normally present in the atmosphere in amounts of $1.82 \times 10^{-3}$, $1.14 \times 10^{-3}$ and $8.7 \times 10^{-6}$, respectively, by % volume. Advanced Inorganic Chemistry, Cotton and Wilkinson (Third Edition, Wiley). Thus, for purposes of the present invention, any amount of noble gas may be used which is greater than that found in atmospheric air. For argon, this amount is preferably at least 1% by volume, and is more preferably in excess of 1% by volume for argon.

For neon, krypton and xenon amounts generally used are at least 0.05% each, by volume. However, it is generally preferred that the amounts of neon, krypton and xenon used be greater than about 0.1% each, by volume.

Thus, in general, any gas, mixtures of gases, liquid or mixtures of liquids may be used in accordance with the present invention as long as they contain an amount of at least one noble gas as described above. Preferably, however, they contain at least the preferred amounts noted above.

In addition to noble gases or noble gas mixtures, any gaseous mixture may be used in accordance with the present invention which contains at least one noble gas. For example, it has been found effective to use unpurified mixtures of gases from air separation plants, such as about 90:10 Kr:Xe, and about 1:1 He: Ne, for example.

Furthermore, atmospheric pressures of from less than one to greater than 1 may be used. In fact, in accordance with the present invention, it has been found acceptable to use quite low pressures such as those near vacuum conditions, such as, for example about $10^{-9}$ torr to very high pressures of about 100 atm or greater. However, in accordance with the present invention, it is generally preferable to use atmospheres ranging from about 0.5 to 20 atm, preferably about from 1 to 10 atm.

Furthermore, quite surprisingly, it has been discovered that the action of noble gases in accordance with the present invention in controlling oxidation of chemicals or chemical preparations occurs maximally in the complete absence of oxidation, but is, nevertheless, observed substantially even in the presence of oxygen.

In fact, excellent results are achieved when the chemicals or chemical preparations to be stabilized are prepared under an inert or more preferably a noble gas, atmosphere in the absence of oxygen.

In accordance with the present invention, the effective stabilization is observed even when the noble gas-containing atmosphere contains other inert gases, such as nitrogen. The effect appears to be directly related to the actual quantitative amount of noble gas present and available to the reactive sites on the subject chemical or chemical preparation.

In general, in accordance with the present invention, the effect thereof is observed even when the noble gas-containing atmosphere contains other reactive gases including oxygen, carbon dioxide, water, or nitrous oxide. In such cases, diminution of the antioxidative effect is seen in direct consequence of the inclusion of these oxygen-containing gases, and is a direct, linear function of their concentration. Thus, the maximum antioxidative effect of argon is seen when argon is present as 100% of the superimposed atmosphere (and effectiveness may be increased by increasing the relative molar concentration of argon by increasing pressure), whereas the minimal effect is observed when argon is present in concentrations only slightly greater than its normal concentration in air (0.1% by volume) admixed with any of oxygen, carbon dioxide, water or nitrous oxide. Generally, the method is most effective when noble gas concentrations in excess of 50% of the atmosphere are used, preferably in excess of 90%, preferably in excess of 95% of the atmosphere.

Furthermore, the improvement of the present invention is generally noted for noble gas-containing atmospheres which might also contain other gases, except that reactive components of these other gases may promote oxidation and mask the desired effect. Thus, complex mixtures of gases which include noble gases will promote oxidation less than similar mixtures which do not contain noble gases.

In general, in accordance with the present invention, the antioxidation potential of the noble gases is of the order of:

$$Xe > Kr > Ar > Ne > He$$

However, this order is strongly dependent upon the actual atmosphere present, particularly the presence of oxygen-containing or other oxidizing species, on the presence of reducing species, and on the nature of the oxidation reaction itself.

Moreover, the observed effectiveness of the noble gases is partially, but not wholly dependent, upon their relative solubility in the chemicals or chemical preparations treated.

Additionally, the observed effectiveness of the noble gases is partially, but not wholly dependent, upon their relative ability to desorb oxygen or water from subject chemical preparations.

Further, the effects of the noble gases may be either ameliorated or potentiated by the admixture of other gases thereto.

Moreover, the observed improvement in accordance with the present invention is notable for noble-containing solutions, either aqueous or non-aqueous, when applied to retard oxidation, in a manner similar to the use of atmospheres. In this use, the optimal method consists of saturating the solution with the desired gas by sparging or superimposition or by use of pressure or through introduction of cryogenic liquid.

In accordance with the present invention, the storage of chemicals or chemical preparations under cryogenic liquid composed of or containing noble gases, such as cryogenic argon, show marked improvement in storage characteristics and retardation of oxidation compared with cryogenic nitrogen (inert) or carbon dioxide (reactive).

Generally, in accordance with the present invention, the quantative improvement in shelf-life may be observed is several times that observed with nitrogen, with the improvement being 25 to 100% being obtained with ease.

Furthermore, the preferred method of use of the present invention is to store the chemicals or chemical preparations under an atmosphere containing a noble gas, mixture of noble gases or gaseous mixture containing at least one noble gas which gases exhibit the best effect, and additionally storing in gas- and moisture-impermeable containers, storing at low temperatures and storing in containers which block incident light or other radiation.

Generally, any chemical or chemical preparation which is susceptible to oxidative degradation may be protected by the present invention. For example, such chemicals or preparations may include chemical reagents, deoxygenated solvents, reducing agents and catalysts. Further, storage temperatures of from about -20°C to about 60°C, preferably from about -10°C to about 30°C, may be used.

Having described the present invention, the same will now be further illustrated by reference to certain examples which are provided solely for purposes of illustration and are not intended to be limitative.

EXAMPLES

A wide range of chemicals has been assayed for oxidative degradation under test atmospheres. These chemical preparations were chosen to be representative (but are not to be considered exclusive) of the various commercially valuable chemical preparations which are subject to oxidative degradation.

The progress of the oxidations were monitored either by ultraviolet/visible spectrophotometry, or by spectrocolorimetry, or by direct visual observation, or by the production of a detectable product (detected chemically, or by gas chromatography/mass spectrometry, or by thin layer chromatography), or by other standard means, depending upon the nature of the chemical oxidation to be measured.

The atmospheres tested include argon, xenon, krypton, neon, helium, nitrogen, oxygen, air, carbon dioxide, nitrous oxide, and decile mixtures of each possible pair and triad, and certain representative other combinations. Tests were conducted in the presence or absence of water vapor, light, temperature, and under differing conditions of concentration and pH. In certain cases, atmospheres were changed after a certain time period in order to observe changing effects. For instance, it was observed in every case of noble gas retardation of oxidation that replacing the noble gas-containing atmosphere with air or oxygen immediately produced the maximum degenerative effect, and that replacing the effective noble gas-containing atmosphere with nitrogen immediately ended the observed retardation effect.

Generally, nitrous oxide, carbon dioxide, air and oxygen produced degenerative oxidative effects, nitrogen diminished these effects to the extent that oxygen was excluded from the reaction, and the noble gases diminished the effect further. This further effect was evident even when oxygen was added in amounts greater than could be accounted for by the different desorptive ability of the various noble gases and nitrogen, and in amounts greater than could be accounted for by different solubilities of the gases in solution.

Pressures tested varied from near vacuum to 100 atm, effectiveness of the noble gases increases with pressure, whereas nitrogen does not show increased effectiveness.

Examples of compounds presently stored under nitrogen which have been found to be more stable when stored under the present gases are:

Quercetin
Rutin
Apomorphine HCl
Morphine sulfate
Epinephrine free base
Epinephrine HCl
Quinine hemisulfate salt
Tetracycline HCl
Retinol
Salicylic acid
L-ascorbic acid
Essential oils
Acetylcholine
Dimercaprol
Ergotamine
Ergonovine
Phenylbutazone
Turbocaine
Vitamin B1

Specific Examples:

Example 1: Quercetin

OXIDATION OF SENSITIVE CHEMICALS
QUERCETIN (Sigma 0-0125)

Part I.

. Solution preparation: Solution C

Dissolve 0.01 g of Quercetin (Sigma Q-0125) into 100 ml of 0.05 M TRIS buffer pH 9.0 (25°C). Solution initial color: yellow
Stored in refrigerator in a Nalgene amber bottle.

. Gas saturation:

Transfer 7 ml/ea. of solution C into 2 10cc serum vials. Cap and seal (rubber stopper + aluminum seal).
Bubble oxygen (on-line) through 1st vial for 1 min and leave under positive pressure. Label vial C/$O_2$.
Bubble nitrogen (on-line) through 2nd vial for 1 min and leave under positive pressure. Label vial C/$N_2$.

· Storage:

The 2 vials are stored at room temperature over the week-end (unprotected from light).

PART II.

1. Visual observations: 09/14/91

Vial C/$O_2$ (see 09/13/91 QUERC01.WP) has turned orange, while vial C/$N_2$ is still yellow. No further color changes are observed on the following days.

2. Full Range Scans (900-190 nm): 09/16/91

Prepare 2 gas-tight acrylic cuvets by stoppering them with blue silicone.
Purge cuvet 1 with $O_2$ on-line for 30 s. Purge cuvet 2 with $N_2$ on-line for 30 s.
Blank cuvet: acrylic cuvet filled with 3 ml D.I. $H_2O$.
Using a 3cc syringe previously purged with $O_2$, transfer 3 ml from vial C/$O_2$ into cuvet 1.
Using a 3cc syringe previously purged with $N_2$ transfer 3 ml from vial C/$N_2$ into cuvet 2.
Spectrophotometer PARAM:

Absorbance
Slit 1 nm
Speed 1,500 nm/min
Resp 1

Asave Y
Aprt N

Full range scans (900-190 nm) vs blank:

Cuvet 1      Filename CO201.SP
Cuvet 2      Filename CN201.SP

A comparison of CO201.SP and CN201.SP spectra shows that oxidation of quercetin causes an increase in ABS at 325 nm and a decrease in ABS at 400 nm. It is therefore decided to monitor the oxidation of quercetin by following the decrease in ABS at 400 nm.

Part III

1. Solution Preparation:

Prepare a fresh quercetin solution by dissolving 10 mg of Quercetin (Sigma Q-0125) into a 100 ml of 0.05 M TRIS buffer pH 9.0 (25°C). Transfer solution to a 100cc serum vial. Cap and seal (rubber stopper + aluminum seal). Flush solution with on-line nitrogen (20 psi delivery pressure) for 3 min. Leave vial under positive pressure. Wrap vial with aluminum foil to prevent light degradation. Store in refrigerator: solution C.

2. CPRG 5 Cells at 400 nm: RUN 1

Blank cuvet: acrylic cuvet filled with 2.5 ml of TRIS buffer pH 9.0.
Label and stopper 5 acrylic cuvets with blue silicone (gas-tight). Label = QT4Gn, where:

Q = quercetin
T4 = trial no. 4
G = gas
n: 1 = $O_2$; 2 = $N_2$; 3 = Ar; 4 = Kr; 5 = Xe

Flush cuvets with on-line nitrogen for 1 min/each. Leave under two 10cc syringes.
Purge a 3cc syringe with $N_2$. Sample 2.5 ml of solution C from 100cc serum vial. Inject into QT4G2 cuvet. Proceed in the same manner for QT4G3,4,5, and 1. The order in which the cuvets are mentioned is respected.
Bubble 2x10cc of the appropriate gas into each cuvet. Remove 10cc syringes. Add 4cc of the appropriate gas in each cuvet to leave it under positive pressure. The gassing is done in the increasing order of gases solubility in $H_2O$: $N_2$, Ar, Kr and Xe. $O_2$ is done last to avoid that too much oxidation of quercetin occurs before starting the spectrophotometric run.
Immediately after gassing, place cuvets in cell holder (Temp.: 25°C). Start run.

CPRG 5 cells
Wavelength 400 nm

Data points 450 ====> 2 HR run
Int. 16 s

Filenames: QT4G1...5.SP

3. CPRG 5 Cells at 400 nm: RUN 2

Same procedure as for Run 1 except for the gases that are run:

QT4G6.SP $O_2$
QT4G7.SP $N_2$
QT4G8.SP Ar
QT4G9.SP Ne
QT4G0.SP Air

The gassing is done in the following order: $N_2$, Ne, Ar, Air and finally $O_2$.

4. CPRG 5 Cells at 400 nm: RUN 3

Same procedure as for Run 1 except for the following:

· Prepare a freshly new quercetin solution by dissolving 10 mg of Quercetin (Q-0125) into a 100 ml of 0.05 M TRIS buffer pH 9.0. Store in refrigerator in a Nalgene amber bottle: SOLUTION IS KEPT IN AIR. NO $N_2$ FLUSHING.
· No $N_2$ flushing of acrylic cuvets: initial headspace is air.

The gassing order is still: $N_2$, Ar, Kr, Xe and finally $O_2$.
Filenames: QT5G1...5.SP

5. CPRG 5 Cells at 400 nm: RUN 4

Same procedure as for Run 3 except f or the gases that are run:

QT5G6.SP $O_2$
QT5G7.SP $N_2$
QT5G8.SP Ar
QT5G9.SP Ne
QT5G0.SP Air

The gassing is done in the following order: $N_2$, Ne, Ar, Air and finally $O_2$.

Part IV

1. Solution preparation:

Weigh out 10 mg of Quercetin (Sigma Q-0125). Put into a 100cc serum vial. Cap and seal (rubber stopper + aluminum seal). Flush vial headspace with on-line nitrogen (very low flow) for 3 min.

Fill a 100cc serum vial with 110 ml of 0.05 M TRIS buffer pH 9.0 (25°C). Cap and seal. Bubble thoroughly with on-line nitrogen (high flow) for 5 min to displace $O_2$. Leave under positive pressure.
Remove 2x50cc of $N_2$-flushed TRIS buffer from vial using a 60cc B-D syringe (purged with $N_2$ prior to sampling) and inject into the 100cc serum vial containing the quercetin dry chemical: solution C.
Bubble the quercetin solution with on-line $N_2$ for 5 min. Leave under positive pressure.
The 100 µg/ml quercetin solution is stored in refrigerator (serum vial being wrapped in aluminum foil to prevent light degradation).

2. CPRG 5 Cells at 400 nm: RUN 1

Blank cuvet: acrylic cuvet filled with 2.5 ml of TRIS buffer pH 9.0.
Label and stopper 5 acrylic cuvets with blue silicone (gas-tight). Label QO2T1G0n, where:

Q = quercetin
$O_2$ = $O_2$ addition
T1 = trial no. 1
G = gas
n: 1 = $O_2$; 2 = Air; 3 = $N_2$; 4 = Ar; 5 = Xe

Flush cuvets with on-line nitrogen for 1 min/each. Leave under two 10cc syringes.
Using 3cc B-D syringes (purged with $N_2$), transfer 2.5 ml of solution C into each cuvet. Bubble 4x10cc of the appropriate gas into each cuvet (except for air and $O_2$) in the following order: $N_2$, Ar, and then Xe.
Fill 5 tuberculin syringes with 1 ml of $O_2$/each.
Then, operating as fast as possible, bubble 4x10cc of air in QO2T1G02 cuvet. Bubble 4x10cc of $O_2$ in QO2T1G01 cuvet. Place the cuvets into cell holder (temp. 25°C). Pick needles of tuberculin syringes into blue silicone stoppers of cuvets (after purging 0.5 ml of $O_2$ out). Slide needles through and push plungers simultaneously, thus injecting 0.5 ml of $O_2$ per cuvet. Start run.

CPRG 5 cells
Wavelength 400 nm
Data points 450 ====> 2 HR run
Int. 16 s

Filenames: QO2T1G01...5.SP

3. CPRG 5 Cells at 400 nm: RUN 2

Same procedure as for Run 1 except for the following:

· Gassing: 2x10cc of the appropriate gas only (instead of 4x10cc) in order to reduce the time lag between each cuvet gassaturation (especially for air

and $O_2$).

· Purge out 0.75 ml of $O_2$ from tuberculin syringes: injection of <u>0. 25 ml of $O_2$</u> per cuvet.

Filenames QO2T2G01...5.SP

Part V.

LONG TERM STORAGE EXPERIMENT

1. Solution preparation:

Weigh out 10 mg of Quercetin (Sigma Q-0125). Put into a 100cc serum vial. Cap and seal (rubber stopper + aluminum seal). Flush vial headspace with on-line nitrogen (very low flow) for 3 min.

Fill a 100cc serum vial with 110 ml of 0.05 M TRIS buffer pH 9.0 (25°C). Cap and seal. Bubble thoroughly with on-line nitrogen (high flow) for 5 min to displace $O_2$. Leave under positive pressure.

Remove 2x50cc of $N_2$-flushed TRIS buffer from vial using a 60cc B-D syringe (purged with $N_2$ prior to sampling) and inject into the 100cc serum vial containing the quercetin dry chemical: solution C.

Bubble the quercetin solution with on-line $N_2$ for 5 min. Leave under positive pressure.

The 100 µg/ml quercetin solution is stored in refrigerator (serum vial being wrapped in aluminum foil to prevent light degradation).

2. Time zero sample:

Blank cuvet: acrylic cuvet filled with 2.5 ml of TRIS buffer pH 9.0.

Stopper 1 acrylic cuvet with blue silicone (gas-tight). Flush cuvet with on-line nitrogen for 1 min. Using a 3cc B-D syringe (purged with $N_2$), transfer 2.5 ml of solution C into the cuvet.

Run a full range scan (900-190 nm) of sample cuvet vs blank.

Spectrophotometer PARAM:

Absorbance
Slit 1 nm
Speed 1,500 nm/min
Resp 1

Asave Y
Aprt N

Filename: QUERINIT.SP

3. Vial Preparation for Long Term Storage:

Cap and seal 7 10cc serum vials (rubber stopper + aluminum seal). Flush vials thoroughly with on-line nitrogen for 2 min/each. Leave under two 10cc syringes.

Using a 10cc syringe (purged with $N_2$ prior to sampling), remove 7 ml of solution C from the 100cc vial and inject into a 10cc vial. Proceed in the same manner for the remaining 10cc vials.

Bubble 3x30cc of the appropriate gas through each vial. Inject an additional 10cc of the appropriate gas to leave vial under positive pressure.

Gassing is done in the following order: $N_2$, Ne, Ar, Kr, Xe, Air, and finally $O_2$.

The 10cc vials are left on the lab bench at room temperature.

4. Full Range Scans (900-190 nm) :

The vials are regularly observed visually for color changes.

After a 30 day-period:

· Blank cuvet: acrylic cuvet filled with 2.5 ml of TRIS buffer pH 9.0.

· Label and stopper one acrylic cuvet: QUED30G1 (QUE = quercetin; D30 = 30 day-period; G1 = Air). Flush cuvet with on-line $N_2$ for 1 min. Then flush cuvet with 4x10cc of the appropriate gas (air). Leave under two 10cc syringes.

· Using a 3cc syringe (purged with the appropriate gas prior to sampling), remove 2.5 ml of air-saturated quercetin solution and inject into cuvet. Scan (900-190 nm).

· Proceed in the same manner for the other vials.

Spectrophotometer PARAM:

Absorbance
Slit 1 nm
Speed 1,500 nm/min
Resp 1
Asave Y
Aprt N

Filenames:

QUED30G1.SP Air
QUED30G2.SP $N_2$
QUED30G3.SP Ar
QUED30G4.SP Kr
QUED30G5.SP Xe
QUED30G6.SP Ne
QUED30G7.SP $O_2$

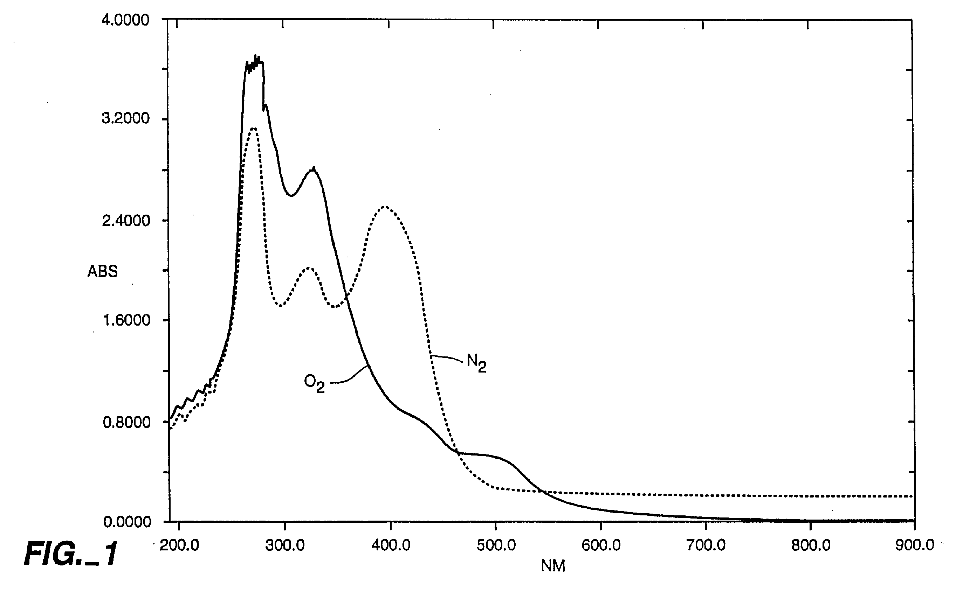

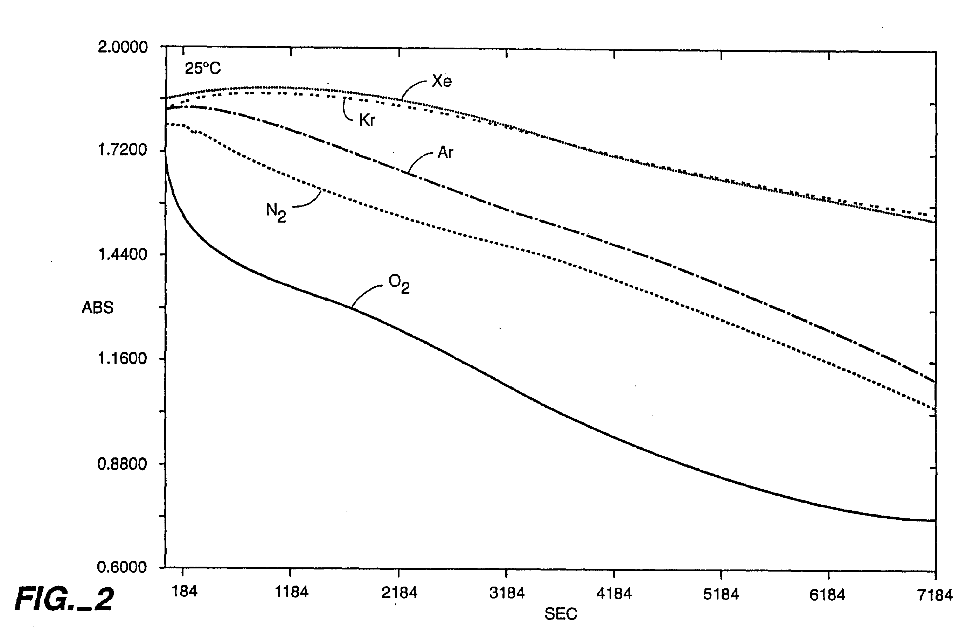

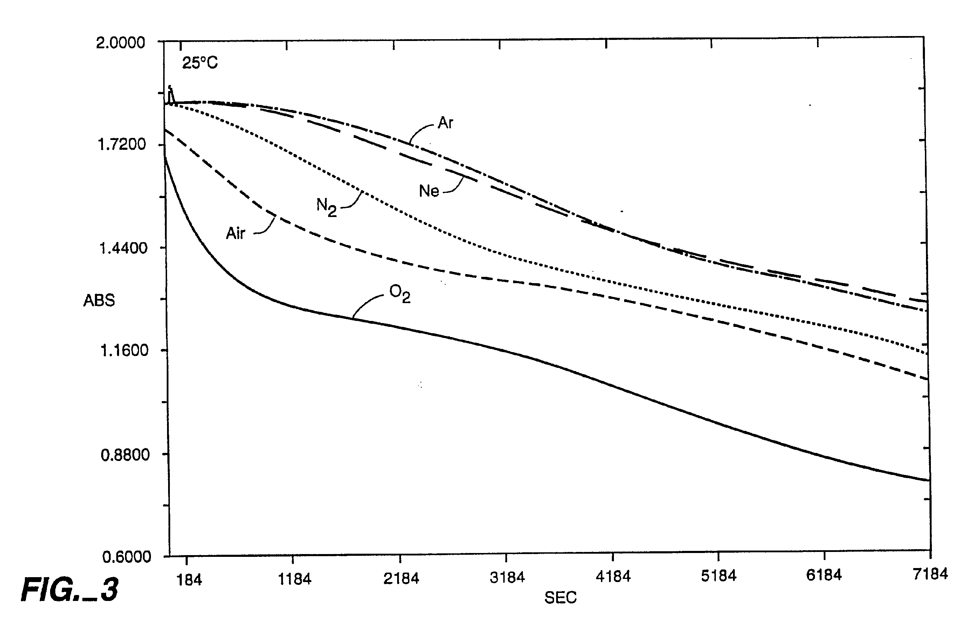

Results for the quercetin experiments are summarized in Figures 1-3. Figure 1 shows that following ABS at 400 nm will reveal the progressive oxidation of the molecule. Figure 2 shows that nitrogen retards oxidation poorly compared to argon, and krypton and xenon inhibit oxidation much better than argon. Figure 3 shows a replicate (differences between Figs. 2 and 3 are due to different start times) which shows additionally that neon inhibits about as well as argon. Since the solubilities of argon and neon are very different yet that of neon and nitrogen very similar, the activity is clearly at least partly independent of solubility.

Example 2: Morphine sulfate salt

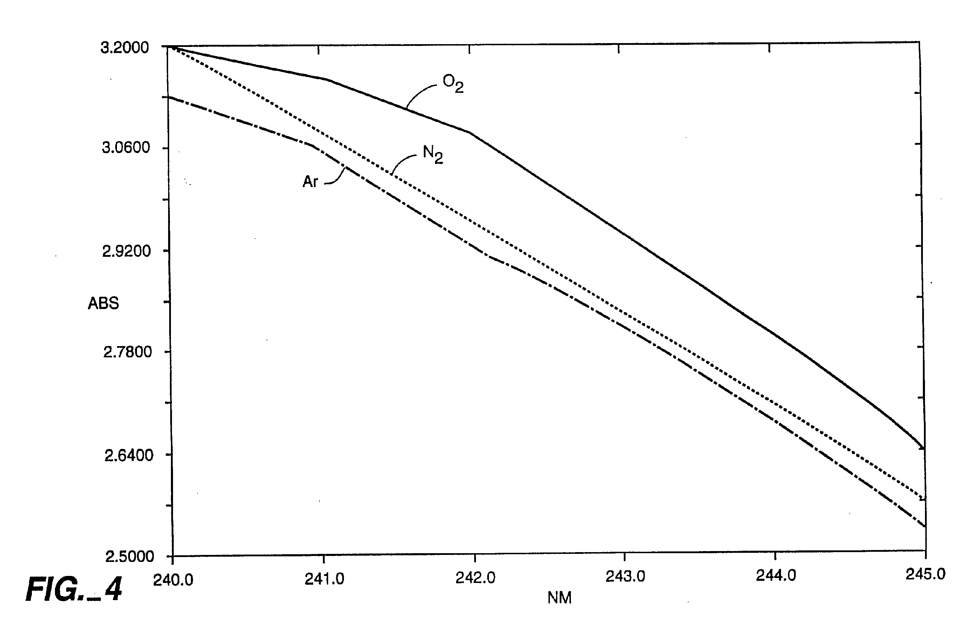

Stored as a solution of 0.1 mg/ml in methanol prepared under nitrogen in the dark f or 31 days. Results in Figure 4 shows the difference in oxidation in the absorbance range 220-260 nm where increased absorbance is proportional to increased oxidation.

Example 3 Apomorphine HCl

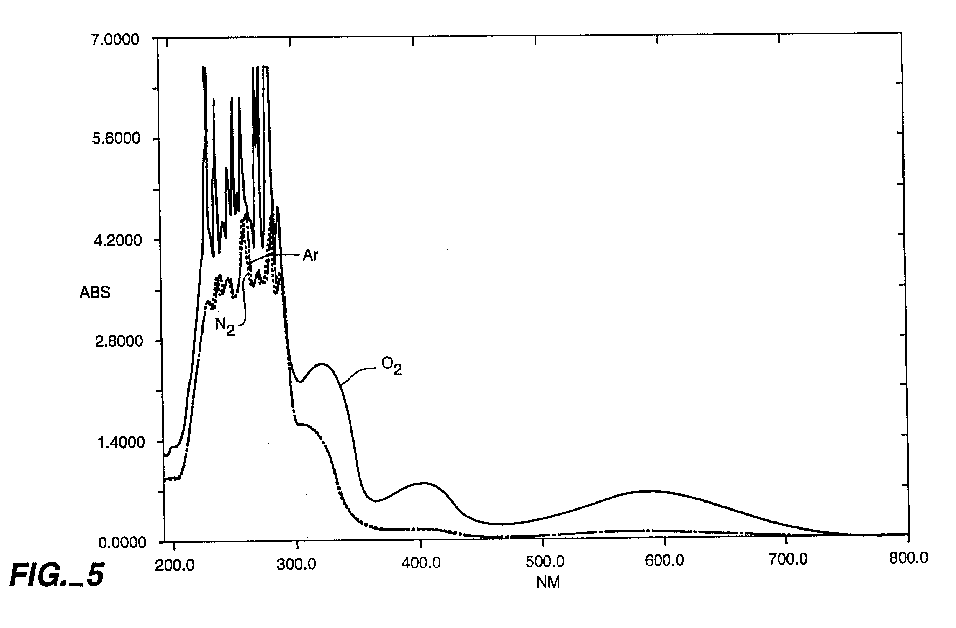

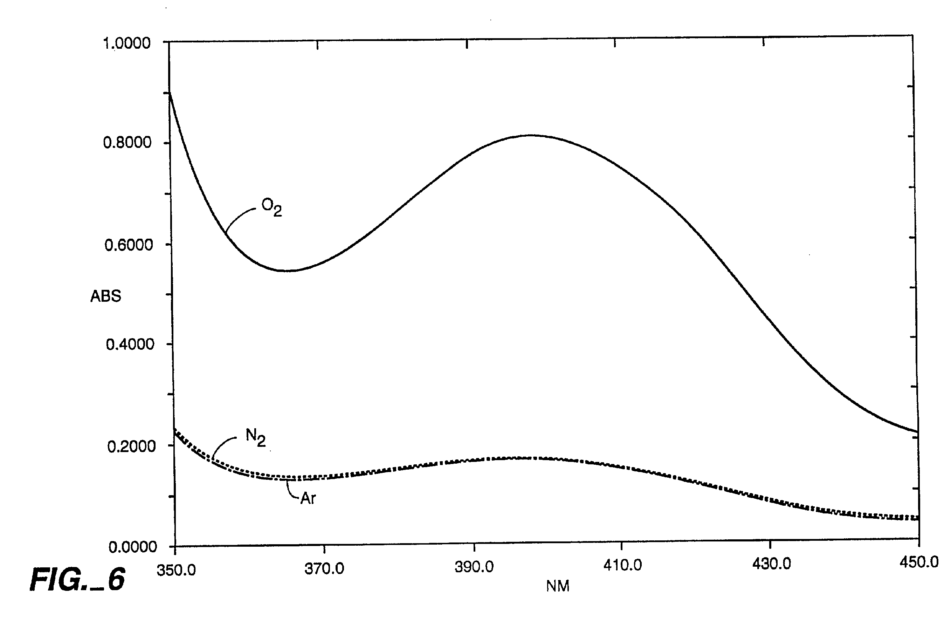

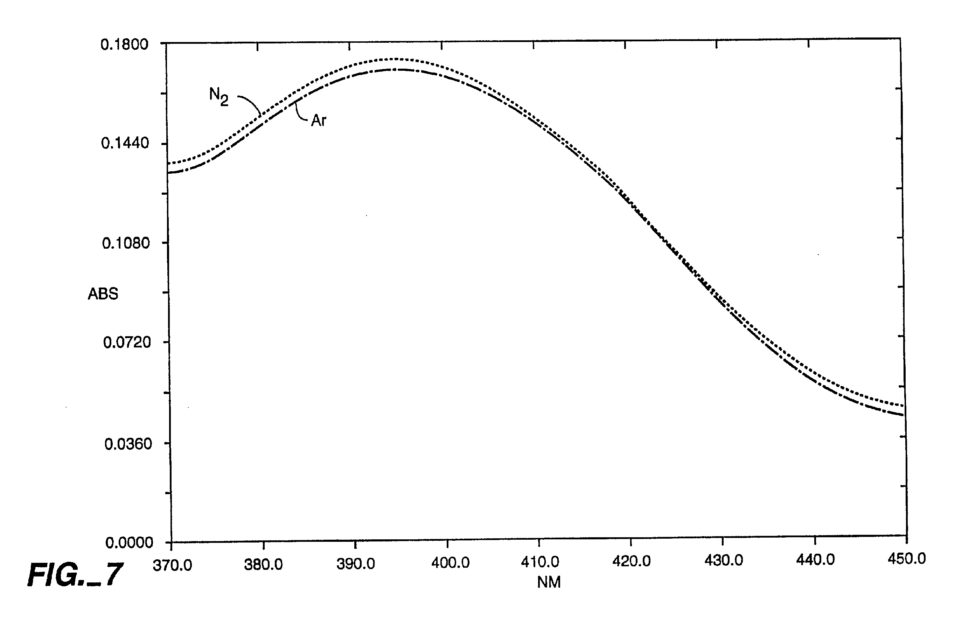

Stored as a solution of 0.1 mg/ml in methanol, this compound readily oxidizes to become green in color. This portion of the spectrum is shown after storing for 31 days under oxygen, nitrogen or argon in three consecutive Figures. Figure 5 shows the total spectrum, identifying the large increase in absorbance after 300 nm caused by oxidation. Figure 6 shows the critical region of 370 - 450 nm, and the maximal oxidation caused by oxygen. Figure 7 shows the significant difference between argon and nitrogen after 31 days. After 120 days the difference between the oxygen and nitrogen samples becomes zero, whereas the argon absorbance is one-half that of oxygen.

Example 4 Epinephrine HCl

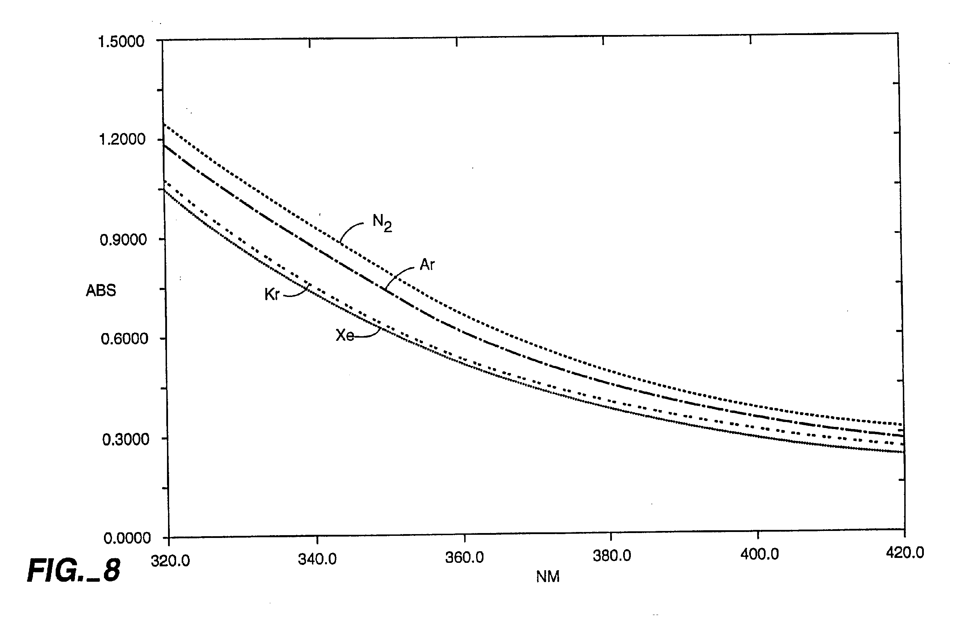

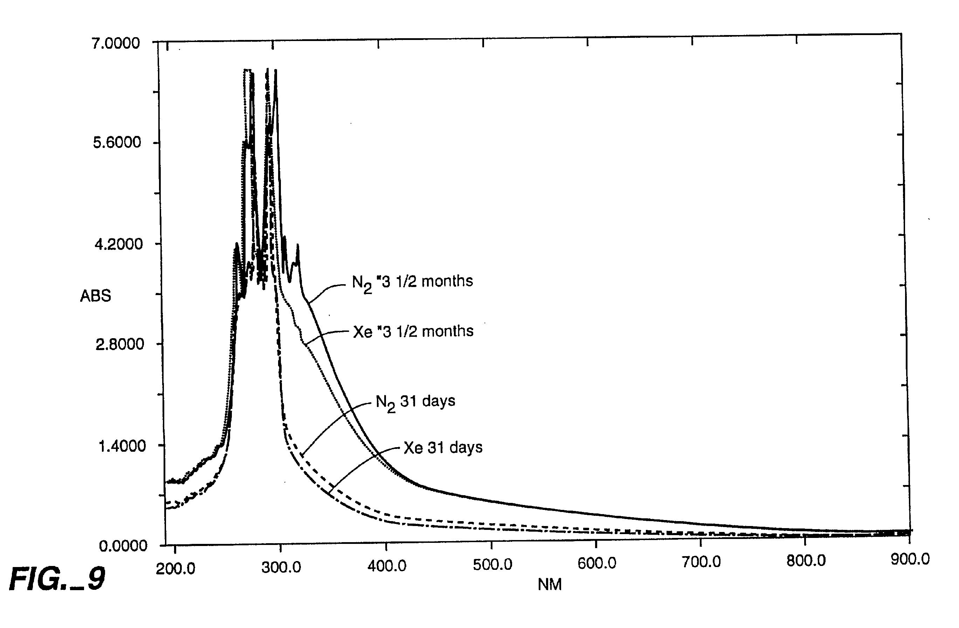

Stored as a solution in degassed water as 40 mg/ml and assayed monthly for 12 months. Figure 8 demonstrates the superiority of xenon and argon over nitrogen in preventing oxidation measured as full scan absorbance abscissal shifts (increasing = oxidized form). Figure 9 demonstrates the same for nitrogen and xenon in comparing degree of oxidation after 1 month versus 3 1/2 months of storage.

Example 5: L-Ascorbic acid

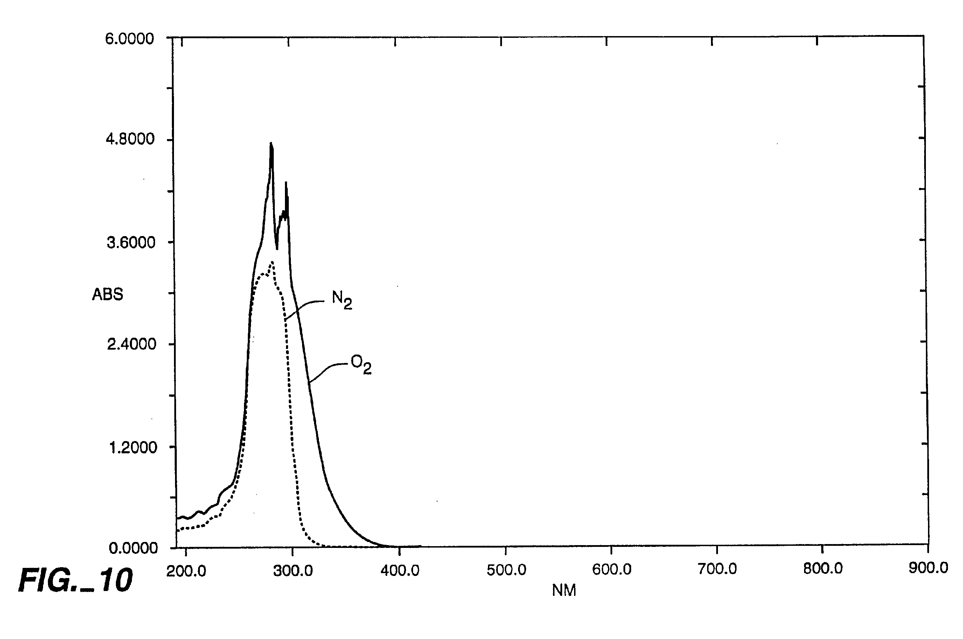

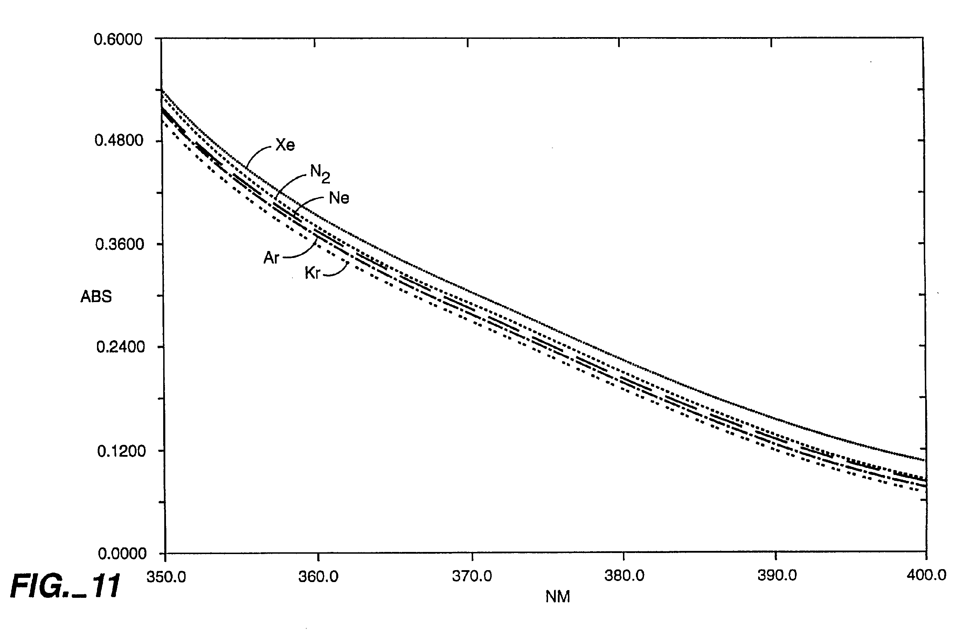

Stored as a solution of 100 mg/ml in degassed water. Figure 10 shows the oxidative abscissal absorbance shift. Figure 11 shows that preservation is in the order $Kr>Ar>Ne>N_2>Xe$ after 30 days.

Example 6: Retinol

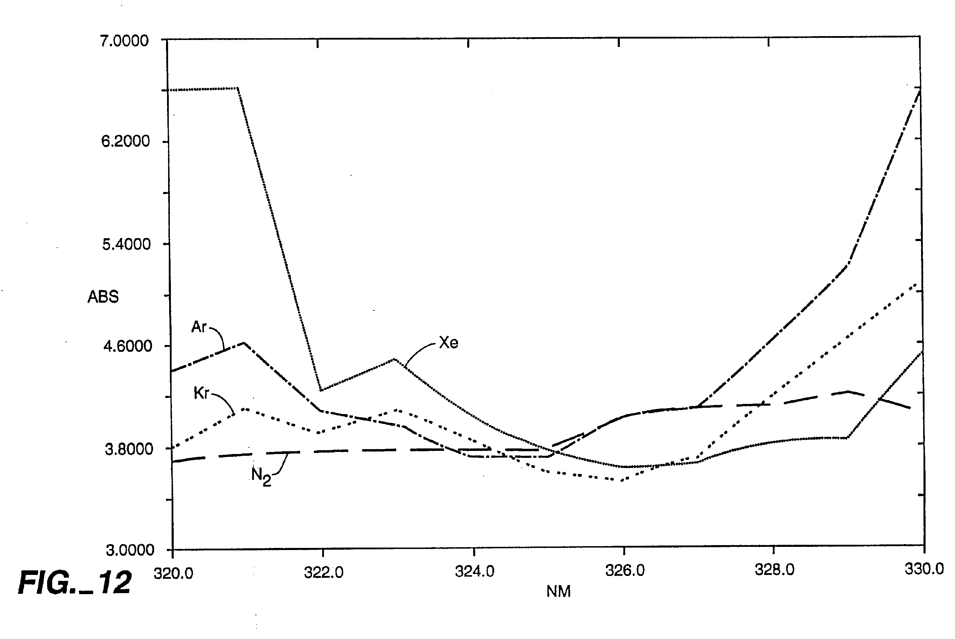

Figure 12 shows the very great improvement obtained in storing retinol under noble gases compared with nitrogen. Here, diminution of absorbance in the 320-324 nm range is indicative of oxidation.

Example 7: Tetracycline HCl

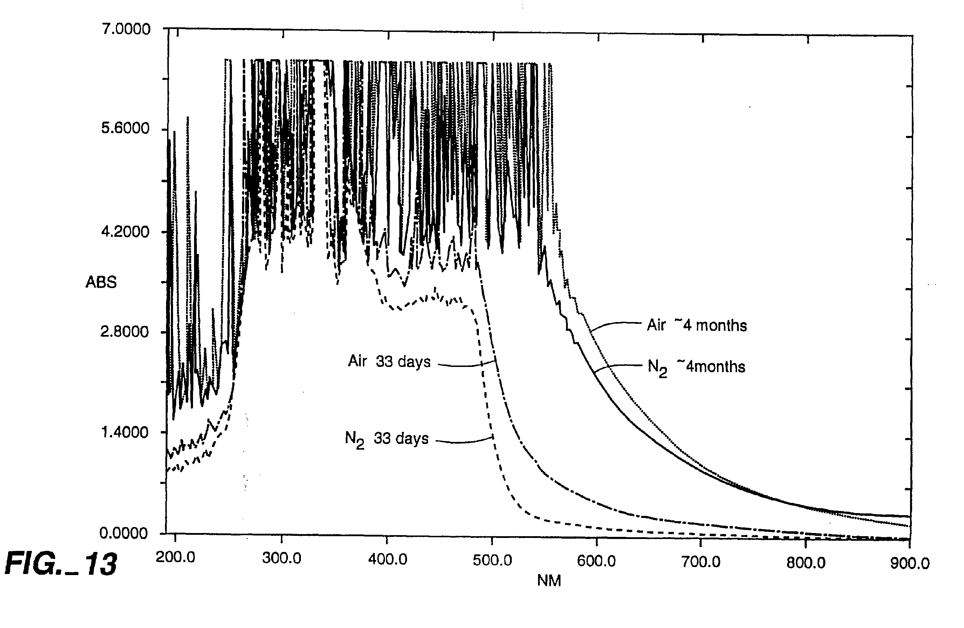

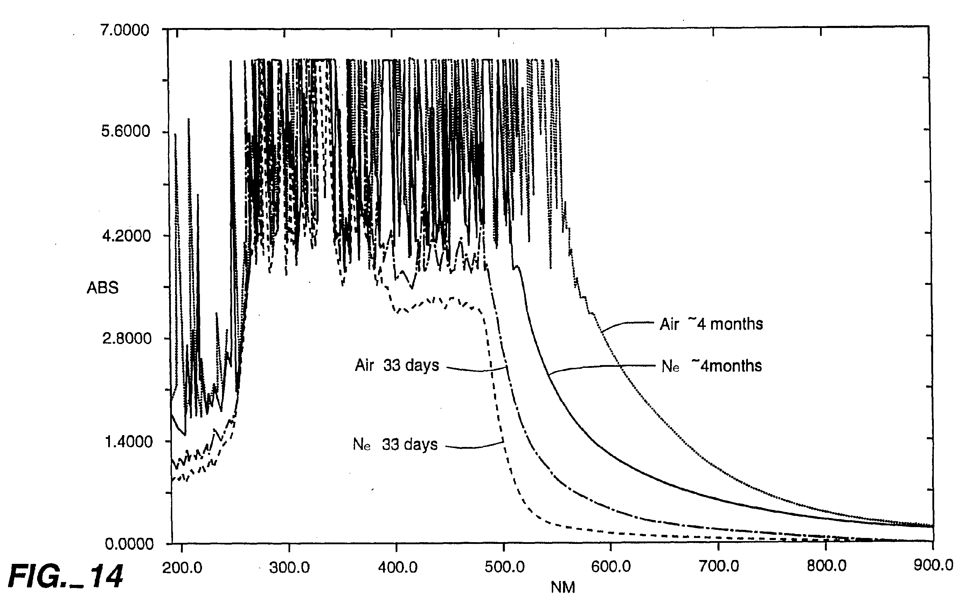

Stored as 10 mg/ml in water for 1 and 4 months. Figure 13 compares oxidation over this time period in an oxygen atmosphere versus a nitrogen atmosphere. Comparing Figure 13 with Figure 14 demonstrates clearly that a neon atmosphere provides greater protection.

Thus, the present invention is surprisingly effective in controlling oxidation of chemicals or chemical preparations or both in storage.

Generally, lower and ambient temperature are preferable for storage, and the same temperature ranges conventionally used for the storage of chemicals and chemical preparations may be used in accordance with the present invention.

Further, while binary mixtures, such as argon-krypton, krypton-neon, xenon-krypton or argon-neon, for example, ternary mixtures or even quaternary mixtures of noble gases may be used.

Also, in addition to offstream gases, such as about 1:1, He/Ne and about 90:10 Kr/Xe, it is also advantageous to use a mixture containing at least one noble gas in deoxygenated air. As used herein, the term "deoxygenated" generally means an oxygen content of less than about 15%, better yet less than about 10%, and more preferably less than about 5% by volume.

Further, the gases or gas mixture may be introduced either in gaseous or liquid form. If liquid form, the noble gases may be used neat or dissolved in a suitable liquid gas such as liquid nitrogen.

Furthermore, it is understood that the advantage of the present invention may be obtained even if the chemicals or chemical preparations are contacted with the gases of the present invention for only a portion of the storage period. However, it is generally preferably to effect such contact throughout the entire storage period.

Having described the present invention, it will now be apparent to one of ordinary skill in the art that many changes and modifications can be made to the various embodiments described above without departing from the spirit and the scope of the present invention.

**Claims**

1. A method of controlling oxidation of a non-enzyme containing chemical or a non-enzyme containing chemical preparation during storage, said chemical or chemical preparation being in the form of a solution, **characterized by** sparging into the solution an atmosphere containing a noble gas selected from the group consisting of argon, krypton, neon, xenon and mixtures thereof to saturation, said atmosphere being used at a pressure of up to and including 10 atmospheres, and wherein said chemical or chemical preparation is selected from the group consisting of Quercitin, Rutin, Apormorphine Hcl, Morphine sulfate, Epinephrine free base, Epinephrine Hcl, Quinine hemisulfate salt, Tetracycline Hcl, Retinol, Salicylic acid, L ascorbic acid, Essential oils, Acetylcholine Dimercaprol, Ergotamine, Ergonovine, Phenylbutazone, Turbocaine and Vitamin B1.

2. The method according to claim 1, wherein said atmosphere being used at a pressure ranging from 1 to 10 atmospheres.

3. The method according to claim 1 or 2, wherein said atmosphere being about 90/10 Kr:Xe.

4. The method according to claim 1 or 2, wherein said atmosphere comprises neon.

FIG._1

FIG._2

EP 1 258 522 A2

FIG._3

FIG._4

FIG._5

FIG._6

EP 1 258 522 A2

**FIG._7**

EP 1 258 522 A2

FIG._8

FIG._9

EP 1 258 522 A2

FIG._10

FIG._11

FIG._12

FIG._13

EP 1 258 522 A2

FIG._14

EP 1 258 522 A2